(19) Europäisches Patentamt
European Patent Office
Office européen des brevets

(11) Veröffentlichungsnummer : **0 119 961**
**B1**

(12) # EUROPÄISCHE PATENTSCHRIFT

(45) Veröffentlichungstag der Patentschrift : **20.01.88**

(51) Int. Cl.[4] : **C 07 D 211/46**, C 08 K 5/34

(21) Anmeldenummer : **84810121.8**

(22) Anmeldetag : **15.03.84**

(54) **Oligoester mit Polyalkylpiperidingruppen.**

(30) Priorität : **21.03.83 CH 1513/83**

(43) Veröffentlichungstag der Anmeldung : **26.09.84 Patentblatt 84/39**

(45) Bekanntmachung des Hinweises auf die Patenterteilung : **20.01.88 Patentblatt 88/03**

(84) Benannte Vertragsstaaten : **DE FR GB IT**

(56) Entgegenhaltungen :
**EP-A- 0 002 005**
**EP-A- 0 002 260**
**EP-A- 0 016 723**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Karrer, Friedrich, Dr.**
**Rebbergstrasse 5**
**CH-4800 Zofingen (CH)**
Erfinder : **Rody, Jean, Dr.**
**Rütiring 82**
**CH-4125 Riehen (CH)**

EP 0 119 961 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft oligomere Ester aus Dicarbonsäuren und Dialkoholen, die mindestens eine Polyalkylpiperidingruppe enthalten, sowie die Verwendung dieser Verbindungen als Lichtschutzmittel für organische Materialien.

In der DE-OS 27 19 131 sind bereits Polyester, die Polyalkylpiperidingruppen enthalten und die ein Molekulargewicht ($\bar{M}n$) von etwa 1 000 bis 5 000 aufweisen, als wertvolle Lichtschutzmittel für Kunststoffe beschrieben. Diese polymeren Lichtschutzmittel haben gegenüber niedermolekularen Polyalkylpiperidinderivaten den Vorteil der geringeren Flüchtigkeit und Extrahierbarkeit. Sie werden hergestellt durch Polykondensation annähernd äquimolarer Mengen einer Dicarbonsäure bzw. deren Dialkylester mit einem Diol, wovon mindestens eine der beiden Komponenten einen Polyalkylpiperidinrest enthält. Die Endgruppen dieser Polyester sind die funktionellen Gruppen der Ausgangsmaterialien, also Hydroxylgruppen und Carboxyl- oder Alkylcarboxylat-Gruppen. Ein allgemeiner Nachteil dieser Herstellungsmethode ist die mangelhafte Reproduzierbarkeit des Molekulargewichtes der hergestellten Polyester. Für bestimmte Anwendungszwecke können ausserdem die funktionellen Endgruppen störend wirken.

Es wurde daher in Weiterbildung der erfinderischen Idee der Schaffung von polymeren Lichtschutzmitteln in der EP-A1-2 005 und US 4 234 707 vorgeschlagen, die Endgruppen durch Zusatz einer monofunktionellen Verbindung während oder nach der Herstellung des oligomeren Polyesters zu blockieren und dadurch das Molekulargewicht zu begrenzen und Produkte mit reproduzierbarem Molekulargewicht zu erhalten. Bei Zusatz von 5 bis 20 Mol-% eines Monocarbonsäureesters oder eines Monoalkohols zur Polykondensation werden dabei oligomere Polyester vom Molekulargewicht ($\bar{M}n$) von etwa 2 000 bis 3 000 erhalten. Die nach diesem Verfahren hergestellten beschriebenen oligomeren Polyester sind spröde Harze, die bei relativ niedrigen Temperaturen erweichen.

Für bestimmte Zwecke ist es wünschenswert, Stabilisatoren von noch niedrigerem Molekulargewicht zu haben, die aber noch die Vorteile der niedrigen Flüchtigkeit und Extrahierbarkeit besitzen. Solche Produkte sind z. B. erwünscht für eine schnellere Einarbeitung in die zu stabilisierenden Polymeren. Für bestimmte Zwecke ist es weiterhin wünschenswert, flüssige Stabilisatoren zu haben, beispielsweise für die Verwendung in Lacken. Solche extrem niedermolekularen Polykondensationsprodukte lassen sich aber nach den oben geschilderten Herstellungsverfahren unter Verwendung annähernd äquimolarer Mengen Dicarbonsäure und Diol nicht mehr in reproduzierbarer Weise herstellen.

Es wurde gefunden, dass sich Polyalkylpiperidingruppen enthaltende Polyester von extrem niedrigen Polykondensationsgrad in einfacher Weise dadurch herstellen lassen, dass man bei ihrer Herstellung durch Polykondensation aus einem Dicarbonsäureester und einem Diol eine der beiden Komponenten in einem molaren Ueberschuss von 33 bis 100 % einsetzt und gegebenenfalls die erhaltenen Oligoester durch Reaktion mit einer den Endgruppen entsprechenden Menge einer monofunktionellen Verbindung nachbehandelt.

Man erhält dabei Produkte, die die gewünschte Eigenschaftskombination von guter Löslichkeit im Substrat und geringer Flüchtigkeit und Extrahierbarkeit besitzen. Diese Oligoester entsprechen den Formeln I oder II

$$E\!-\!\!\left(O\!-\!R^1\!-\!O\!-\!\overset{O}{\overset{\|}{C}}\!-\!R^2\!-\!\overset{O}{\overset{\|}{C}}\right)_{\!n}\!-\!O\!-\!R^1\!-\!O\!-\!E \qquad (I)$$

$$E'\!-\!\!\left(\overset{O}{\overset{\|}{C}}\!-\!R^2\!-\!\overset{O}{\overset{\|}{C}}\!-\!O\!-\!R^1\!-\!O\right)_{\!n}\!-\!\overset{O}{\overset{\|}{C}}\!-\!R^2\!-\!\overset{O}{\overset{\|}{C}}\!-\!E' \qquad (II)$$

worin n ein Wert von 1 bis 3 ist, $R^1$ und $R^2$ zweiwertige organische Reste mit den folgenden Bedentungen sind, von denen mindestens einer einen Polyalkylpiperidinrest der Formel III enthält,

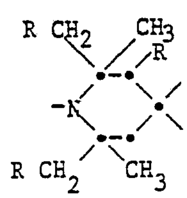

(III)

worin R Wasserstoff oder $C_1$-$C_4$-Alkyl ist, und E und E′ Endgruppen sind.

Wie bei jeder Polykondensation sind die entstehenden Produkte keine einheitlichen Verbindungen sondern Gemische von Verbindungen mit verschiedenem Polykondensationsgrad. Der angegebene Wert n ist daher ein Mittelwert und ist meistens keine ganze Zahl. In den entstehenden Oligoester-Gemischen kann jedoch eine Verbindung mit n = 1 oder 2 oder 3 in dominierender Menge vorhanden sein, wenn das

Verhältnis der Ausgangskomponenten entsprechend gewählt wird.

Verwendet man als Ausgangskomponente zwei verschiedene Diole oder die Diester von zwei verschiedenen Dicarbonsäuren, so erhält man Co-oligoester der Formel I oder II, worin die Gruppen $R^1$ oder $R^2$ untereinander verschieden sind. Auch diese Co-oligoester sind Gegenstand der Erfindung.

Die Endgruppen E in Formel I sind entweder Wasserstoff, dann handelt es sich um Oligoester-diole. Werden diese mit einer monofunktionellen Verbindung nachbehandelt, so wird das Radikal dieser monofunktionellen Verbindung zur Endgruppe E. Analog verhält es sich mit den Endgruppen E'. Diese sind ohne Nachbehandlung Alkoxygruppen und es handelt sich bei den Produkten (der Formel II) um Oligoester-diester. Werden diese mit monofunktionellen Verbindungen nachbehandelt, so wird der Rest der monofunktionellen Verbindung zur Endgruppe E'.

$R_1$ und $R_2$ haben die folgenden Bedeutungen : $R^1$ $C_2$-$C_{18}$-Alkylen, $C_4$-$C_8$-Alkenylen, $C_4$-$C_{20}$-Mono- oder Polyoxaalkylen, $C_5$-$C_8$-Cycloalkylen, $C_7$-$C_{10}$-Cycloalkan-dialkylen, $C_8$-$C_{20}$-Aren-dialkylen oder einen zweiwertigen Rest der Formeln

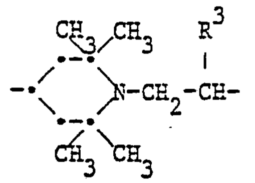

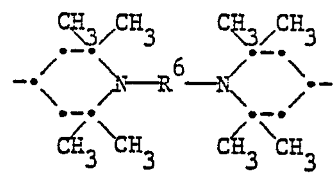

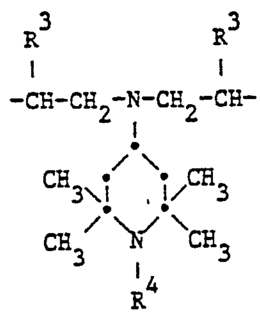

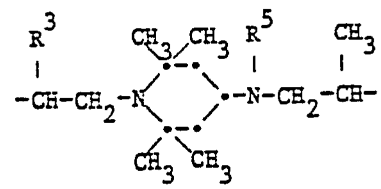

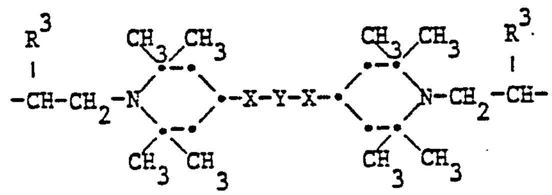

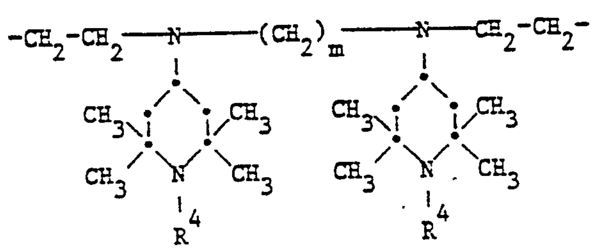

oder

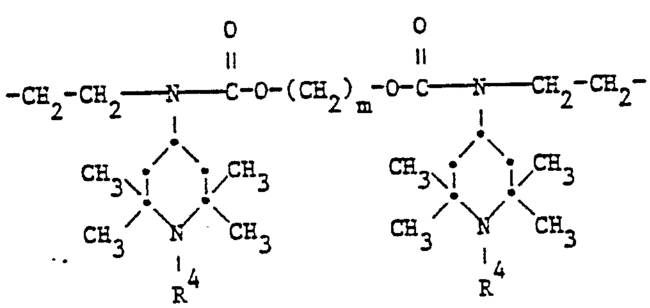

bedeutet, worin m 2 bis 10 ist,

X eine Gruppe —O— oder —$NR^5$— ist,

Y $C_2$-$C_{12}$-Alkylen, durch ein oder zwei —O— oder —NH— unterbrochenes $C_4$-$C_{12}$-Alkylen, Cycloalkylen, $C_6$-$C_{12}$-Arylen, oder -Phenylen-Z-Phenylen- bedeutet, worin

Z —O—, —$CH_2$— oder —$SO_2$— ist,

$R^2$ eine direkte Bindung, $C_1$-$C_{18}$-Alkylen, $C_2$-$C_6$-Alkenylen, $C_5$-$C_8$-Cycloalkylen, $C_5$-$C_8$-Cycloalkenylen, $C_6$-$C_{12}$-Arylen, $C_7$-$C_{12}$-Aralkylen, oder einen zweiwertigen Rest der Formeln

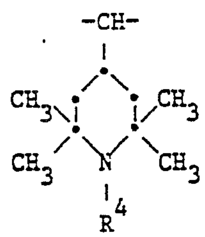
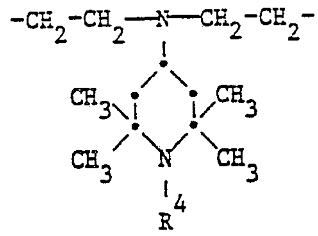

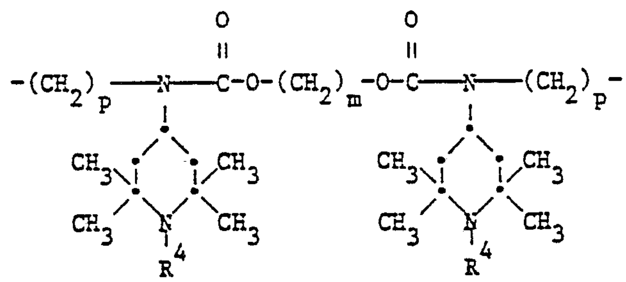

bedeutet, worin p 1 oder 2 ist,

E Wasserstoff, $C_1$-$C_8$-Alkyl, Cyclohexyl, Benzyl oder eine Acylgruppe der Formel $R^7$—CO—, $R^8$—O—CO— oder ($R^9$)($R^{10}$)N—CO— und

E' eine Gruppe $R^{11}$O— oder ($R^{12}$)($R^{13}$)N— darstellt,

worin

$R^3$ Wasserstoff, $C_1$-$C_6$-Alkyl, $C_2$-$C_7$-Alkoxymethyl, Phenoxymethyl oder Tolyloxymethyl bedeutet

$R^4$ Wasserstoff, $C_1$-$C_8$-Alkyl, $C_2$-$C_8$-Alkanoyl, $C_3$-$C_6$-Alkenoyl, $C_7$-$C_{12}$-Phenylalkyl oder $C_3$-$C_5$-Alkenylmethyl bedeutet,

$R^5$ Wasserstoff, $C_1$-$C_{12}$-Alkyl, Cyclohexyl, Phenyl, Benzyl, Phenylethyl, $C_2$-$C_8$-Alkanoyl, $C_3$-$C_6$-Alkenoyl oder Benzoyl bedeutet,

$R^6$ $C_2$-$C_{12}$-Alkylen, $C_4$-$C_8$-Alkenylen oder Xylylen bedeutet,

$R^7$ $C_1$-$C_{12}$-Alkyl, $C_2$-$C_5$-Alkenyl, $C_5$-$C_8$-Cycloalkyl, $C_6$-$C_{10}$-Aryl, $C_7$-$C_{12}$-Phenylalkyl oder eine Gruppe der Formel IV bedeutet,

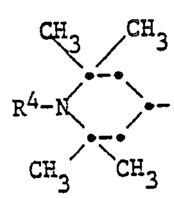

(IV)

$R^8$ $C_1$-$C_{12}$-Alkyl, Allyl, Cyclohexyl oder Phenyl bedeutet,

$R^9$ Wasserstoff, $C_1$-$C_8$-Alkyl, Allyl, Cyclohexyl, Phenyl oder $C_7$-$C_{10}$-Alkylphenyl und

$R^{10}$ $C_1$-$C_8$-Alkyl, Allyl, Cyclohexyl, Phenyl oder $C_7$-$C_{10}$-Alkylphenyl bedeuten oder $R^9$ und $R^{10}$ zusammen mit dem N-Atom, an das sie gebunden sind, einen 5- bis 7-gliedrigen heterocyclischen Ring bilden,

$R^{11}$ $C_1$-$C_{12}$-Alkyl, $C_3$-$C_5$-Alkenylmethyl, $C_5$-$C_8$-Cycloalkyl, Phenyl, $C_7$-$C_{10}$-Alkylphenyl, $C_7$-$C_{12}$-Phenylalkyl, $C_3$-$C_{12}$-Alkoxyalkyl, eine Gruppe —$(CH_2CH_2O)_m$—$CH_3$, eine Gruppe der Formel IV oder eine Gruppe der Formel V bedeutet,

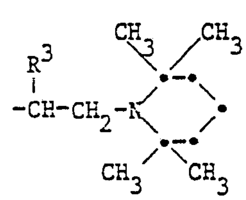

(V)

$R^{12}$ $C_1$-$C_{12}$-Alkyl, Allyl, Cyclohexyl, Phenyl, Benzyl, $C_3$-$C_{12}$-Alkoxyalkyl, $C_4$-$C_{12}$-Dialkylaminoalkyl, eine Gruppe der Formel IV oder der Formel VI

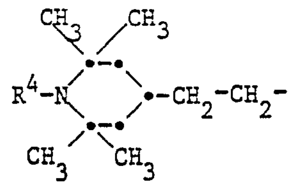

(VI)

bedeutet und

$R^{13}$ Wasserstoff, $C_1$-$C_{12}$-Alkyl, Allyl, Cyclohexyl oder eine Gruppe der Formel IV bedeutet, sowie deren Co-oligomere.

$R^1$ kann darin unverzweigtes oder verzweigtes $C_2$-$C_{18}$-Alkylen sein wie z. B. 1,2-Ethylen, 1,3-Propylen, 1,2-Propylen, Tetra-, Penta-, Hexa-, Octa-, Deca-, Dodeca-, Tetradeca- oder Octadecamethylen, 3-Methyl-1,3-butylen, 2,5-Hexylen, 2-Ethyl-1,3-hexylen, 2,2-Dimethyl-1,3-hexylen oder 2,2-Dimethyl-1,3-propylen.

$R^1$ als $C_4$-$C_8$-Alkenylen kann insbesondere Alkenylen-dialkylen sein, wie z. B. 2-Butenylen-1,4, 3-Hexenylen-2,5 oder 4-Octenylen-3,6.

$R^1$ als $C_4$-$C_{20}$-Mono- oder Polyoxaalkylen kann z. B. 3-Oxapentylen-1,5, 3,6-Dioxaoctylen-1,8, 4-Oxaheptylen-2,6 oder der zweiwertige Rest eines technischen Polyethylenglykolgemisches $-(CH_2CH_2O)_r-CH_2-CH_2-$ mit r = 2 bis 19, insbesondere mit r = 2 bis 9 sein.

$R^1$ als Cycloalkylen oder Cycloalkan-dialkylen kann z. B. 1,3-Cyclopentylen, 1,3- oder 1,4-Cyclohexylen, 1,5-Cyclooctylen, Cyclohexan-1,3-dimethylen, Cyclohexan-1,4-dimethylen oder Bicyclo [2.2.1] heptan-2,3-dimethylen sein. $R^1$ als Aren-dialkylen kann z. B. m- oder p-Xylylen oder Diphenyl-4,4'-dimethylen sein.

Y als $C_2$-$C_{12}$-Alkylen kann unverzweigtes oder verzweigtes Alkylen sein, wie z. B. Di-, Tri-, Tetra-, Hexa-, Octa-, Deca-, Dodecamethylen, 1,2-Propylen oder 2,2-Dimethyl-1,2-propylen. Y als durch O oder NH unterbrochenes Alkylen kann z. B. 3-Azapentylen-1,5, 4-Azaheptylen-1,7 4-Oxaheptylen-1,7, 3,6-Diazaoctylen-1,8 oder 4,11-Diaza-tetradecylen-1,14 sein. Y als $C_6$-$C_{12}$-Arylen kann z. B. 1,3- oder 1,4-Phenylen, 1,4- oder 1,5-Naphthylen oder 4,4'-Diphenylen sein.

$R^2$ als $C_1$-$C_{18}$-Alkylen kann unverzweigtes oder verzweigtes Alkylen sein, wie z. B. Methylen, Di-, Tri-, Tetra-, Hexa-, Octa- oder Dodecamethylen, Propylen-1,2, Pentylen-3,3 oder 2-Methylpropylen-1,3. $R^2$ als $C_2$-$C_6$-Alkenylen kann z. B. Vinylen, Propenylen-1,2 oder Propenylen-1,3 sein.

$R^2$ als Cycloalkylen oder Cycloalkenylen kann z. B. Cyclopentylen-1,3,Cyclohexylen-1,4, Cyclohexylen-1,3,-4-Cyclohexenylen-1,2 oder Bicyclo [2.2.1] hepten-5-ylen-2,3 sein. $R^2$ als Arylen oder Aralkylen kann z. B. 1,3- oder 1,4-Phenylen, 1,4- oder 1,5-Naphthylen, 4,4'-Diphenylen, 1,3-Xylylen oder 1,4-Xylylen sein.

$R^6$ als $C_2$-$C_{12}$-Alkylen kann insbesondere unverzweigtes Alkylen sein, wie z. B. Di-, Tri-, Tetra-, Hexa- oder Octamethylen. $R^6$ als Alkenylen kann insbesondere 2-Butenylen-1,4 sein.

$R^3$ als $C_1$-$C_6$-Alkyl kann z. B. Methyl, Ethyl, Propyl, n-Butyl, sec.-Butyl, iso-Amyl oder n-Hexyl sein. $R^4$, $R^9$, $R^{10}$ und E als $C_1$-$C_8$-Alkyl können darüber hinaus auch z. B. n-Heptyl, 2-Ethylhexyl oder n-Octyl sein. $R^5$, $R^7$, $R^8$, $R^{11}$, $R^{12}$, $R^{13}$ als $C_1$-$C_{12}$-Alkyl können darüber hinaus auch z. B. Isononyl, n-Decyl oder n-Dodecyl sein.

$R^3$ als $C_2$-$C_7$-Alkoxymethyl kann z. B. Ethoxymethyl, Butoxymethyl oder 2-Ethylbutoxymethyl sein.

$R^{11}$ und $R^{12}$ als $C_3$-$C_{12}$-Alkoxyalkyl können z. B. 2-Methoxyethyl, 2-Isopropoxyethyl, 2-Ethoxopropyl, 2-Butoxyethyl, 2-(2-Ethylhexyloxy)-ethyl, 3-Methoxypropyl oder 3-Ethoxypropyl sein.

$R^{12}$ als $C_4$-$C_{12}$-Dialkylaminoalkyl kann insbesondere 3-Dialkylaminopropyl sein, wie z. B. 3-Dimethylamino- oder 3-Diethylaminopropyl.

$R^7$ als $C_2$-$C_5$-Alkenyl kann z. B. Vinyl, Propenyl-2, 2-Propenyl-1, 1-Propenyl-3 oder 2-Methyl-1-propenyl-1 sein. $R^4$ und $R^{11}$ als $C_3$-$C_5$-Alkenylmethyl können z. B. Allyl, Methallyl oder Dimethylallyl sein.

$R^4$, $R^7$, $R^{11}$ als $C_7$-$C_{12}$-Phenylalkyl können z. B. Benzyl, 2-Phenylethyl, 2-Phenylpropyl, 2-Methyl-2-phenylethyl oder 3-Phenylpropyl sein.

$R^7$ und $R^{11}$ als $C_5$-$C_8$-Cycloalkyl können z. B. Cyclopentyl, Cyclohexyl oder Cyclooctyl sein. $R^7$ als $C_6$-$C_{10}$-Aryl kann Phenyl oder Naphthyl sein. $R^9$, $R^{10}$ und $R^{11}$ als $C_7$-$C_{10}$-Alkylphenyl können z. B. Tolyl, Dimethylphenyl, tert.-Butylphenyl oder Di(isopropyl)phenyl sein.

$R^4$ und $R^5$ als $C_2$-$C_8$-Alkanoyl können z. B. Acetyl, Propionyl, Butyryl, Isobutyroyl, Hexanoyl oder 2-Ethylhexanoyl sein. $R^4$ und $R^5$ als $C_3$-$C_6$-Alkenoyl können z. B. Acryloyl, Methacryloyl oder Crotonyl sein.

Wenn $R^9$ und $R^{10}$ zusammen mit dem N-Atom, an das sie gebunden sind, einen 5- bis 7-gliedrigen heterocyclischen Ring bilden, so kann dies z. B. ein Pyrrolidin-, Piperidin-, Morpholin-, 4-Methylpiperazin- oder 4-(2-Cyanoethyl)-piperazinring sein.

Bevorzugt sind solche Oligoester der Formel I oder II, worin n ein Wert von 1 bis 3 ist,

$R^1$ $C_2$-$C_{12}$-Alkylen, $C_4$-$C_{20}$-Mono- oder Polyoxa-alkylen oder eine Gruppe der Formel

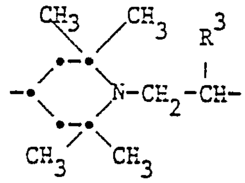

bedeutet,

$R^2$ $C_1$-$C_{12}$-Alkylen, $C_2$-$C_{12}$-Alkenylen oder Phenylen bedeutet,

E Wasserstoff oder eine Gruppe $R^7$—CO— und

E' eine Gruppe $R^{11}O$— ist,

$R^3$ Wasserstoff, $C_1$-$C_4$-Alkyl, $C_2$-$C_6$-Alkoxymethyl, Phenoxymethyl oder Tolyloxymethyl bedeutet,

$R^4$ Wasserstoff, $C_1$-$C_4$-Alkyl, Acetyl, Allyl oder Benzyl bedeutet,

$R^7$ $C_1$-$C_6$-Alkyl oder Phenyl und

$R^{11}$ $C_1$-$C_{12}$-Alkyl oder eine Gruppe der Formel IV oder V bedeutet.

Die Herstellung der Oligoester kann in Lösung oder ohne Lösungsmittel geschehen. Als Lösungsmittel eignen sich insbesondere Kohlenwasserstoffe, wie Toluol, Xylol, Tetralin, Decalin oder Ligroin. Vorzugsweise arbeitet man in Gegenwart eines Umesterungs-Katalysators, wie z. B. eines Tetraalkyltitanates, eines Alkaliamides, -hydrides oder -alkoxides oder eines Dialkylzinnoxides.

Die für die Polykondensation notwendige Reaktionstemperatur beträgt 80 bis 230 °C, vorzugsweise arbeitet man bei 120 bis 180 °C.

Die beiden Komponenten können von Anfang an zusammengemischt werden oder man tropft die im Unterschuss verwendete Komponente im Laufe der Reaktion zu. Während der Reaktion wird der aus dem Ester entstehende Alkohol laufend abdestilliert um das Gleichgewicht der Umesterung auf die Seite des Oligoesters zu verschieben. An Hand der Menge des abdestillierten Alkohols kann man den Fortgang der Reaktion verfolgen. Wenn die berechnete Menge des Alkohols abdestilliert ist, lässt man die Reaktionsmischung abkühlen. Der erhaltene Oligoester kann durch Filtration über Fullererde, Kieselgel oder sonstigen adsorptiven Mitteln gereinigt werden. Er kann aber auch ohne Reinigung verwendet werden oder anschliessend mit einem monofunktionellen Reagens umgesetzt werden.

Sind die Endgruppen des primär erhaltenen Oligoesters Hydroxylgruppen (Formel I, E = H) so kann z. B. als monofunktionelles Reagens ein Alkyl-, Cyclohexyl- oder Benzylhalogenid in Gegenwart einer stöchiometrischen Menge Alkali (z. B. in Form von NaH oder $C_2H_5ONa$) verwendet werden. Hierbei entstehen Ether-Endgruppen (E = Alkyl, Cyclohexyl oder Benzyl). Verwendet man als Reagens ein Monocarbonsäurechlorid, -anhydrid oder einen -alkylester, so erhält man Ester-Endgruppen (E = $R^7$CO—). Verwendet man als Reagens ein Chlorcarbonat $R^8$O—COCl, so erhält man Carbonat-Endgruppen (E = $R^8$O—CO—). Verwendet ein Carbamoylchlorid ($R^9$)($R^{10}$)N—CO—Cl, so erhält man Carbamat-Endgruppen (E = ($R^9$)($R^{10}$)N—CO—). In ähnlicher Weise lassen sich auch andere Reagenzien verwenden, die mit Hydroxylgruppen reagieren können.

Sind die Endgruppen des Oligoesters Alkoxycarbonylgruppen (Formel II, E' = —OAlkyl), so kann man diese z. B. mit einer Hydroxylverbindung $R^{11}$OH umestern oder man setzt sie mit einem primären oder sekundären Amin um und erhält dann als Endgruppen Amidgruppen (E' = ($R^{12}$)($R^{13}$)N—).

Die Reaktion mit einer Verbindung $R^{11}$OH kann auch während der Herstellung des Oligoesters geschehen. In diesem Falle kann die Verbindung $R^{11}$OH entweder vor oder während der Polykondensation zugegeben werden.

Nach den hier angegebenen Verfahren erhält man die gewünschten Oligoester in gut reproduzierbarem Molekulargewicht. Diese Produkte sind wirkungsvolle Lichtschutzmittel für organische Materialien wie z. B. Kosmetika, Oele, Photographische Schichten, insbesondere jedoch für organische Polymere wie sie für Kunststoffe, Lacke, Dichtungsmassen oder Klebstoffe verwendet werden. Beispiele für Polymere die auf diese Weise stabilisiert werden können, sind die folgenden :

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.

2. Mischungen der unter 1) genannten Polymeren, z. B. Mischungen von Polypropylen mit Polyisobutylen.

3. Copolymere von Mono- und Diolefinen untereinander oder mit anderen Vinylmonomeren, wie z. B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Aethylidennorbornen.

4. Polystyrol, Poly-(p-methylstyrol).

5. Copolymere von Styrol oder $\alpha$-Methylstyrol mit Dienen oder Acrylderivaten, wie z. B. Styrol-Butadien, Styrol-Acrylnitril, Styrol-Alkylmethacrylat, Styrol-Maleinanhydrid, Styrol-Acrylnitril-Methylacrylat ; Mischungen von hoher Schlagzähigkeit aus Styrol-Copolymeren und einem anderen Polymer wie z. B. einem Polyacrylat, einem Dien-Polymeren oder einem Ethylen-Propylen-Dien-Terpolymeren ; sowie Block-Copolymere des Styrols, wie z. B. Styrol-Butadien-Styrol, Styrol-Isopren-Styrol, Styrol-Ethylen/

Butylen-Styrol oder Styrol-Ethylen/Propylen-Styrol.

6. Pfropfcopolymere von Styrol, wie z. B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z. B. als sogenannte ABS, MBS, ASA oder AES-Polymere bekannt sind.

7. Halogenhaltige Polymere, wie z. B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid ; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z. B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Block-Copolymere mit Polyethern wie z. B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z. B. von Epoxyacrylaten, Urethan-acrylaten oder Polyester-acrylaten.

24. Alkyldharze, Polyesterharze und Acrylatharze, die mit Melaminharzen, Harnstoffharzen, Polyisocyanaten, oder Epoxidharzen vernetzt sind.

25. Vernetzte Epoxidharze, die sich von Polyepoxiden ableiten, z. B. von Bis-glycidyläthern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z. B. PP/EPDM, Polyamid 6/EPDM oder ABS, PVC/EVA, PVC/ABS, PVC/MBS, PC/ABS, PBTP/ABS.

Von besonderer Bedeutung ist die Stabilisierung von Polyolefinen, Styrolpolymerisaten, Polyamiden, Polyurethanen und von Lackharzen wie Alkyd-, Acryl- und Polyesterharzen.

Die oligomeren Stabilisatoren werden den Polymeren in einer Konzentration von 0,01 bis 5 Gew.-%, berechnet auf das zu stabilisierende Material, zugesetzt. Vorzugsweise werden 0,1 bis 2 Gew.-% der Verbindungen, berechnet auf das zu stabilisierende Material, in dieses eingearbeitet.

Die Einarbeitung kann während oder nach der Polymerisation erfolgen, beispielsweise durch

Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die Oligoester können auch in Form eines Masterbatches, der diese Verbindungen beispielsweise in einer Konzentration von 2,5 bis 25 Gew.-% enthält, den zu stabilisierenden Polymeren zugesetzt werden.

Die Erfindung betrifft daher auch die durch Zusatz von 0,01 bis 5 Gew.-% einer Verbindung der Formel I oder II stabilisierten Polymeren, die gegebenenfalls noch andere bekannte und übliche Zusätze enthalten können. Die so stabilisierten Kunststoffe können in verschiedenster Form angewendet werden, z. B. als Folien, Fasern, Bändchen, Profile oder als Bindemittel für Lacke, Klebemittel oder Kitte.

Als weitere Additive, mit denen zusammen die erfindungsgemäss verwendbaren Stabilisatoren eingesetzt werden können, sind beispielsweise zu nennen :

1. Antioxidantien

1.1. Alkylierte Monophenole
2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone
2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

1.3. Hydroxylierte Thiodiphenylether
2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

1.4. Alkyliden-Bisphenole
2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

1.5. Benzylverbindungen
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat

1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz.

1.6. Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.8. Ester der β-(5-tert.butyl-4-hydroxy-3-methylphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.9. Amide der β-(3,5-Di-tert.butyl-4-hydroxyphenyl)-propionsäure, wie z. B.
N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin

2. UV-Absorber und Lichtschutzmittel

2.1. 2-(2'-Hydroxyphenyl)-benztriazole, wie z. B. das 5'-Methyl-, 3',5'-Di-tert.butyl-, 5'-Tert.butyl-, 5'-(1,1,3,3-Tetramethyl-butyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z. B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z. B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z. B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester,
N-(β-Carbomethoxy-β-cyanovinyl)-2-methyl-indolin,

2.5. Nickelverbindungen, wie z. B. Nickelkomplexe des 2,2'-Thio-bis-[4-(1,1,3,3-tetramethylbutyl)-phenols], wie der 1 : 1- oder der 1 : 2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z. B. Bis-(2,2,6,6-tetramethylpiperidyl)-sebacat Bis-(1,2,2,6,6-pentamethylpiperidyl)-sebacat n-Butyl-3,5-di-tert. butyl-4-hydroxybenzyl-malonsäure-bis (1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert. Octylamino-2,6-dichlor-1,3,5-s-triazin, Tris-(2,2,6,6-tetramethyl-4-piperidyl)-nitrilotriacetat,

Tetrakis-(2,2,6,6-tetramethyl-4-piperidyl)-1,2,3,4-butantetracarbonsäureester, 1,1'-(1,2-Ethandiyl)-bis-(3,3,5,5-tetramethyl-piperazinon).

2.7. Oxalsäurediamide, wie z. B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho- und para-Methoxy- sowie von o- und p-Ethoxy-di-substituierte Oxaniliden.

3. Metalldesaktivatoren, wie z. B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z. B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit.

5. Peroxidzerstörende Verbindungen, wie z. B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z. B. Kupfersalze in Kombination mit Jodiden und/oder Phosphorverbindungen und Salze des zweiwertigen Mangans.

7. Basische Co-Stabilisatoren, wie z. B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z. B. 4-tert.Butylbenzoesäure, Adipinsäure, Diphenylessigsäure.

9. Füllstoffe und Verstärkungsmittel, wie z. B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z. B. Weichmacher, Gleitmittel, Emulgatoren, Pigmente, Optische Aufheller, Flammschutzmittel, Antistatika, Treibmittel.

Bei der Mitverwendung solcher Stabilisatoren können synergistische Effekte auftreten, was besonders bei der Mitverwendung von UV-Absorbern häufig der Fall ist.

Die erfindungsgemässen Oligomeren können auch in Form ihrer Salze verwendet werden. Soweit nicht $R^4$ eine Acylgruppe ist, sind die Piperidingruppen der erfindungsgemässen Oligoester zur Bildung von Salzen mit Säuren befähigt, z. B. mit Salzsäure, Phosphorsäure oder Sulfonsäuren.

Die erfindungsgemässen Copolymeren können auch in Form ihrer Komplexe mit Schwermetallverbindungen, insbesondere Nickel- oder Kobaltverbindungen verwendet werden. Beispiele hierfür sind Komplexe mit Nickelacetat, Nickelstearat oder Nickelacetylacetonat.

Die folgenden Beispiele illustrieren die Herstellung der erfindungsgemässen Oligoester und ihre Verwendung als Lichtschutzmittel. Die darin aufgeführten Temperaturen sind in °C angegeben.

Beispiel 1

Herstellung eines Oligoesters durch Polykondensation im Molverhältnis 3 : 2

Zu einer auf 130-135° erhitzten Lösung von 172,8 g (0,75 Mol) Sebacinsäuredimethylester in 700 ml wasserfreiem Xylol gibt man 1,0 ml Tetrabutyl-orthotitanat. Anschliessend werden in diese Lösung, unter Rühren, innerhalb von 4 Stunden kontinuierlich 100,7 g (0,50 Mol) 1-Hydroxyethyl-4-hydroxy-2,2,6,6-tetramethylpiperidin (HETP) eingetragen. Das durch die fortschreitende Umesterung abgespaltene Methanol — sowie etwas Xylol — werden im schwachen Stickstoffstrom fortwährend ausdestilliert. Nach beendeter Zugabe des Piperidins wird für weitere 5 Stunden bei 135-140° gerührt, wobei das restliche Methanol und Xylol ausdestillieren. Hierauf wird das Xylol im Vakuum vollständig abdestilliert. Der Rückstand wird in Hexan-Diethylether (3 : 1) aufgenommen, mit 25 g Kieselgel und 10 g Tonsil AC versetzt und 30 Minuten bei Raumtemperatur verrührt. Nach der Filtration werden die Lösungsmittel abdestilliert und der Oligoester im Hochvakuum von den letzten Spuren Lösungsmittel befreit.

Der so erhaltene, flüssige Oligoester ist praktisch farblos und besitzt bei 40° eine Viskosität von 1970 cP. Das durch Dampfdruckosmometrie gemessene Molekulargewicht ($\bar{M}n$) beträgt 930. Dies entspricht einem Oligoester der Formel

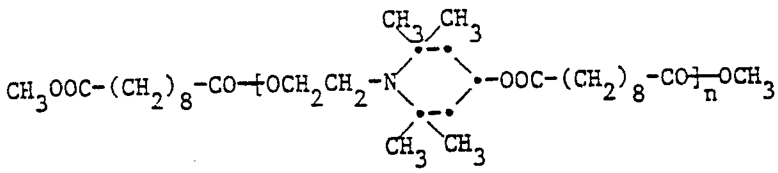

worin n = 1,91 ist.

In analoger Weise wurden die in Tabelle 1 aufgeführten Oligoester aus den angegebenen Diestern und Diolen hergestellt.

(Siehe Tabelle 1 Seite 12 ff.)

Tabelle 1

| Oligoester Nr. | Verwendeter Diester | Diol *) | Molverhältnis Diester: Diol | $\overline{M}n$ | Physik. Eigenschaften |
|---|---|---|---|---|---|
| 1 | $CH_3OOC-(CH_2)_8-COOCH_3$ | HETP | 3:2 | 800 | ölige Flüssigkeit |
| 2 | $C_2H_5OOC-(CH_2)_4-COOC_2H_5$ | HETP | 3:2 | 830 | ölige Flüssigkeit |
| 3 | $C_2H_5$<br>$C_2H_5OOC-C-COOC_2H_5$<br>$C_2H_5$ | HETP | 3:2 | 944 | viskose Masse |
| 4 | $C_4H_9OOC-(CH_2)_4-COOC_4H_9$ | HETP | 3:2 | 855 | ölige Flüssigkeit |
| 5 | $CH_3OOC-(CH_2)_4-COOCH_3$ | HETP | 3:2 | 860 | ölige Flüssigkeit |
| 6 | $CH_3OOC-$ 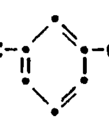 $-COOCH_3$ | HETP | 3:2 | 861 | fest, Ts∼55° |
| 7 | $CH_3OOC-(CH_2)_8-COOCH_3$ | HETP | 4:3 | 1240 | ölige Flüssigkeit |

Tabelle 1 (Fortsetzung)

| Oligoester Nr. | Verwendeter Diester | Diol *) | Molverhältnis Diester: Diol | $\bar{M}n$ | Physik. Eigenschaften |
|---|---|---|---|---|---|
| 8 | 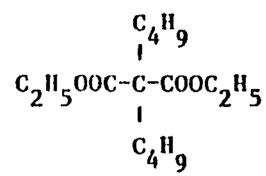 | HPTP | 3:2 | 1030 | ölige Flüssigkeit |
| 9 | 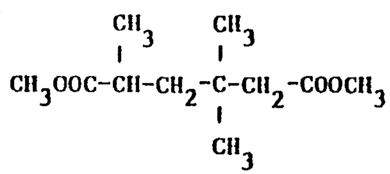 | HPTP | 3:2 | 1160 | ölige Flüssigkeit |
| 10 | $CH_3OOC-(CH_2)_8-COOCH_3$ | HPTP | 3:2 | 910 | ölige Flüssigkeit |
| 11 | $CH_3OOC-(CH_2)_8-COOCH_3$ | HBTP | 3:2 | 950 | ölige Flüssigkeit |
| 12 | $CH_3OOC-(CH_2)_8-COOCH_3$ | BHETP | 3:2 | 920 | ölige Flüssigkeit |
| 13 | 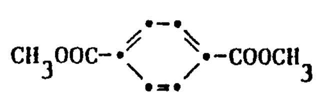 | HETP | 3:2 | 1430 | fest, Ts ca. 180°C |
| 14 | $CH_3OOC-(CH_2)_7-COOCH_3$ | HETP | 3:2 | | ölige Flüssigkeit |

Tabelle 1 (Fortsetzung)

| Oligoester Nr. | Verwendeter Diester | Diol *) | Molverhältnis Diester: Diol | $\bar{M}n$ | Physik. Eigenschaften |
|---|---|---|---|---|---|
| 15 | 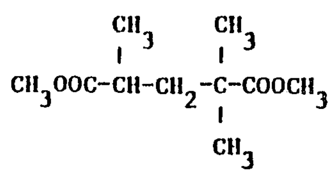 | HETP | 3:2 | 980 | ölige Flüssigkeit |
| 16 | 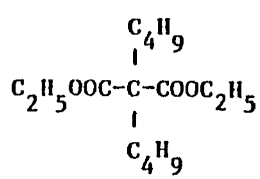 | HETP | 3:2 | | ölige Flüssigkeit |

*) HETP = 1-(2-Hydroxyethyl)-2,2,6,6-tetramethylpiperidinol-4
HPTP = 1-(2-Hydroxypropyl)-2,2,6,6-tetramethylpiperidinol-4
HBTP = 1-(2-Hydroxybutyl)-2,2,6,6-tetramethylpiperidinol-4
BHETP = 1-(3-Butoxy-2-hydroxypropyl)-2,2,6,6-tetramethylpiperidinol-4

Beispiel 2

Herstellung eines Co-oligoesters

55,2 g (0,24 Mol) Dimethylsebacat, 10,4 g (0,06 Mol) Dimethyladipat, 40,3 g (0,20 Mol) HETP und 0,5 ml Tetrabutyltitanat werden in 600 ml wasserfreiem Xylol gelöst und unter Stickstoff 8 Stunden bei 135-140° gerührt unter Abdestillieren des gebildeten Methanols und eines Teiles des Xylols. Dann wird das restliche Xylol im Vakuum abdestilliert und der ölige Rückstand in Methylenchlorid gelöst, die Lösung über Kieselgel filtriert und das Filtrat eingedampft. Die letzten Spuren Lösungsmittel werden im Hochvakuum entfernt. Der hinterbleibende Oligoester ist eine ölige, fast farblose Flüssigkeit und hat gemäss Dampfdruckosmometrie ein Molekulargewicht ($\bar{M}n$) von 930. (Oligoester Nr. 17).

In analoger Weise wird aus 0,2 Mol Dimethylsebacat, 0,1 Mol Di-ethyl-diethylmalonat und 0,2 Mol HEPT ein Co-oligoester als weiche Masse mit einem $\bar{M}n$ von 1170 erhalten (Oligoester Nr. 18).

Beispiel 3

Polykondensation mit anschliessender Umsetzung der Endgruppen.

Zu einer auf 135° erhitzten Lösung von 32,44 g (0,15 Mol) Trimethyladipinsäuredimethylester (2,2,4- und 2,4,4-Trimethyl-Isomerengemisch) in 300 ml wasserfreiem Xylol gibt man 0,2 g Lithiumamid. In diese Lösung werden unter Rühren innerhalb von 4 Stunden portionenweise 20,13 g (0,1 Mol) HETP eingetragen. Das sich abspaltende Methanol wird im schwachen Stickstoffstrom kontinuierlich ausdestilliert. Nach weiteren 6 Stunden Reaktionszeit bei 135-140° ist chromatographisch kein freies HETP mehr nachweisbar. Nun werden 19,93 g (0,1 Mol) 1-Acetyl-4-hydroxy-2,2,6,6-tetramethylpiperidin, 100 ml Xylol und 0,1 g Lithiumamid zugegeben und weitere 7 Stunden bei 140° die Umesterung unter Abdestillieren von Methanol und Xylol im Stickstoffstrom fortgesetzt. Nach 3 Stunden bei 150° wird abgekühlt, das Reaktionsprodukt in Hexan gelöst, mit 8 g Kieselgel 30 Minuten bei ca. 20° verrührt, filtriert und das Lösungsmittel im Vakuum und schliesslich im Hochvakuum vollständig entfernt.

Der so erhaltene Oligoester besitzt bei 120° eine Viskosität von 335 cP. Das mit Hilfe der Dampfdruckmethode gemessene mittlere Molekulargewicht beträgt 1150. Dies entspricht einem Oligoester der Formel

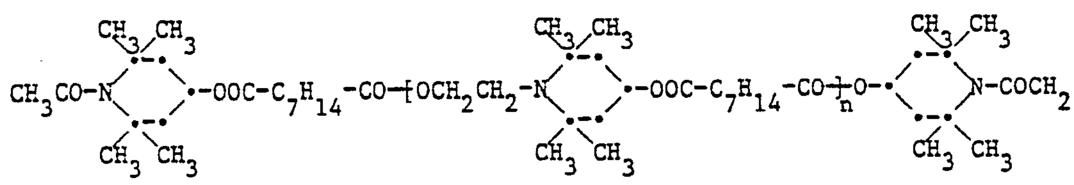

worin n = 1,1 ist.

In analoger Weise wurden die in Tabelle 2 aufgeführten Oligoester aus den angegebenen Komponenten hergestellt.

(Siehe Tabelle 2 Seite 16 f.)

Tabelle 2

| Oligoester Nr. | Diester | Diol | Monofunktionelle Komponente | Molverhältnis | $\bar{M}n$ | Physik. Eigenschaften |
|---|---|---|---|---|---|---|
| 19 | Dimethyl-trimethyl-adipat | HETP | 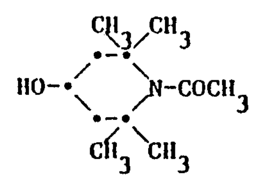 | 3:2:2 | 926 | ölige Flüssigkeit |
| 20 | Diethyl-dibutylmalonat | HETP | 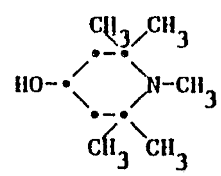 | 3:2:2 | | ölige Flüssigkeit. |
| 21 | Dimethyl-isophthalat | HETP | 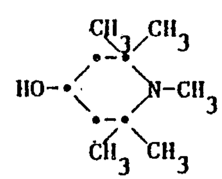 | 3:2:2 | 750 | fest, Ts ca. 68° |

Beispiel 4

Stabilisierung eines 2-Schicht-Metalleffekt-Lackes

Aluminiumbleche von 0,5 mm Stärke werden mit einem Aluminium-pigmentierten Grundlack auf Basis Polyester/Celluloseacetobutyrat/Melaminharz beschichtet. Auf den nassen Grundlack wird ein Klarlack der folgenden Zusammensetzung aufgespritzt :

58,3 Teile Viacryl® VC 373 (Acrylharz der Fa. Vianova, Wien)
27,3 Teile Maprenal® MF 590 (Melaminharz der Fa. Höchst AG, Frankfurt)
1,0 Teil 1 %ige Lösung eines Silikonharzes in Xylol
4,0 Teile Solvesso® 150 (aromatisches Lösungsmittelgemisch)
5,4 Teile Xylol
4,0 Teile Ethylglykolacetat
0,5 Teile Lichtschutzmittel gemäss Tabelle 3.

Die Menge des Lichtschutzmittels entspricht 1 % bezogen auf den Feststoffgehalt des Lackes. Dieser Klarlack hat eine Viskosität von 21 sec/DIN-Becher 4. Er wird in einer Schichtdicke von 40 µm aufgetragen und bei 130 °C 30 Minuten eingebrannt.

Die Proben werden in einem UVCON-Schnellbewitterungsgerät der Fa. Atlas mit einem Zyklus von 4 h UV-Bestrahlung bei 60° und 4 h Bewitterung bei 50° 2 000 h bewittert. Nach 1 000 h und nach 2 000 h wird der 20°-Glanz gemäss DIN 67530 gemessen. Ausserdem werden die Proben in regelmässigen Abständen unter dem Stereomikroskop auf Rissbildung untersucht. Die Ergebnisse sind in Tabelle 3 zusammengestellt.

Tabelle 3

| Lichtschutzmittel | 20°-Glanz nach | | | Rissbildung bemerkbar nach |
|---|---|---|---|---|
| | 0 | 1000 | 2000 h | |
| Keines | 97 | 47 | 9 | 1600 h |
| 1 % Oligoester Nr. 1 | 96 | 65 | 35 | 2600 h |
| 1 % Oligoester Nr. 2 | 96 | 56 | 37 | 2800 h |
| 1 % Oligoester Nr. 10 | 95 | 75 | 29 | 2600 h |
| 1 % Oligoester Nr. 18 | 95 | 61 | 40 | 2600 h |

Beispiel 5

Stabilisierung einer Polypropylen-Folie

100 Teile Polypropylenpulver (Moplen®, Fibre grade, der Firma Montedison) werden mit 0,2 Teilen β-(3,5-Di-tert.-butyl-4-hydroxyphenyl)-propionsäure-octadecylester, 0,1 Teilen Calciumstearat und 0,25 Teilen eines Stabilisators der folgenden Tabelle 1 im Brabender-Plastographen bei 200° während 10 Minuten homogenisiert. Die so erhaltene Masse wird möglichst rasch dem Kneter entnommen und in einer Kniehebelpresse zu einer 2-3 mm dicken Platte gepresst. Ein Teil des erhaltenen Rohpresslings wird ausgeschnitten und zwischen zwei Hochglanz-Hartaluminiumfolien mit einer hydraulischen Laborpresse während 6 Minuten bei 260° zu einer 0,1 mm dicken Folie gepresst, die unverzüglich in kaltem Wasser abgeschreckt wird. Aus dieser werden nun Abschnitte ausgestanzt und im Xenotest 1200 belichtet. In regelmässigen Zeitabständen werden diese Prüflinge aus dem Belichtungsapparat entnommen und in einem IR-Spektrophotometer auf ihren Carbonylgehalt geprüft. Die Zunahme der Carbonylextinktion bei 5,85 µm während der Belichtung ist ein Mass für den photooxidativen Abbau des Polymeren (s. L. Balaban et al., J. Polymer Sci, Part C ; 22 (1969), 1059-1071) und ist erfindungsgemäss mit einem Abfall der mechanischen Eigenschaften des Polymeren verbunden. Als Mass der Schutzwirkung gilt die Zeit bis Erreichen einer Carbonylextinktion von 0,25, bei welcher die Vergleichsfolie brüchig ist.

Zur Prüfung der Flüchtigkeit und der Wasser-Extrahierbarkeit wurde a) ein Teil der Proben vor der Belichtung 7 Tage im Umluftofen auf 120° erwärmt und b) ein anderer Teil der Proben vor der Belichtung 7 Tage lang mit Wasser von 90° behandelt.

Tabelle 4 zeigt die Ergebnisse ohne und mit Vorbehandlung der Proben.

Tabelle 4

| Stabilisator Verbindung Nr. | Belichtungszeit bis zu Carbonylextinktion von 0,25 (in Stunden) | | |
|---|---|---|---|
| | ohne Vorbehandlung | 7 Tage 120° | 7 Tage Wasser-extraktion bei 90° |
| 6 | 4230 | 3750 | 2360 |
| 21 | 5300 | 5010 | 1340 |
| ohne | 1100 | 1070 | 1120 |

**Patentansprüche**

1. Oligomere Ester der Formel I oder II,

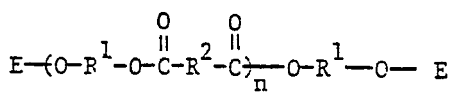

(I)

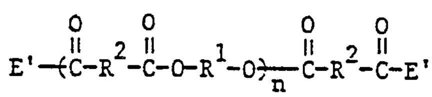

(II)

worin

n ein Wert von 1 bis 3 ist,

$R^1$ und $R^2$ zweiwertige organische Reste sind, von denen mindestens einer einen Polyalkylpiperidinrest der Formel III enthält,

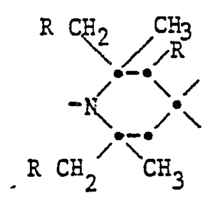

(III)

worin R Wasserstoff oder $C_1$-$C_4$-Alkyl ist, wobei $R^1$ $C_2$-$C_{18}$-Alkylen, $C_4$-$C_8$-Alkenylen, $C_4$-$C_{20}$-Mono- oder Polyoxaalkylen, $C_5$-$C_8$-Cycloalkylen, $C_7$-$C_{10}$-Cycloalkan-dialkylen, $C_8$-$C_{20}$-Aren-dialkylen oder einen zweiwertigen Rest der Formeln

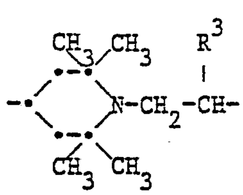

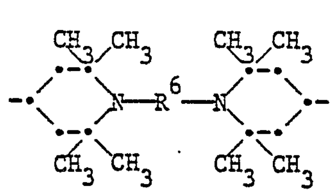

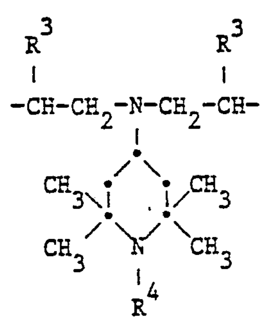

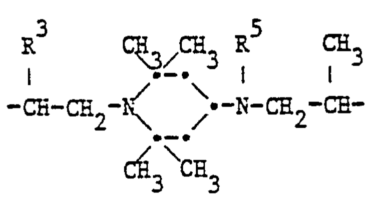

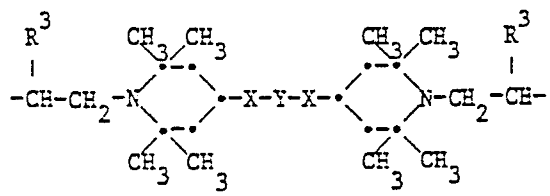

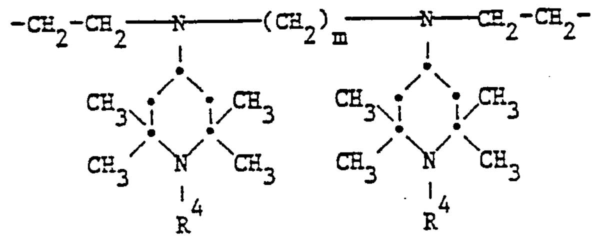

oder

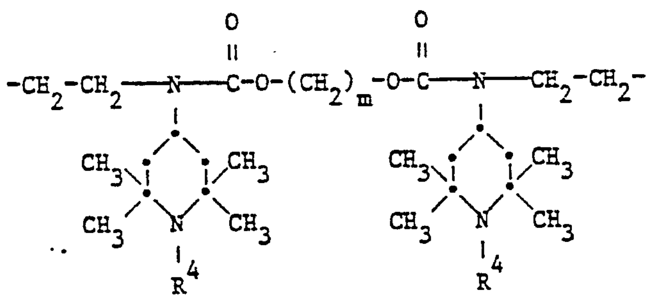

bedeutet, worin

m 2 bis 10 ist,

X eine Gruppe —O— oder —$NR^5$— ist,

Y $C_2$-$C_{12}$-Alkylen, durch ein oder zwei —O— oder —NH— unterbrochenes $C_4$-$C_{12}$-Alkylen, Cyclohexylen, Cyclohexan-dimethylen, $C_6$-$C_{12}$-Arylen, oder -Phenylen-Z-Phenylen- bedeutet, worin

Z —O—, —$CH_2$— oder —$SO_2$— ist,

$R^2$ eine direkte Bindung, $C_1$-$C_{18}$-Alkylen, $C_2$-$C_6$-Alkenylen, $C_5$-$C_8$-Cycloalkylen, $C_5$-$C_8$-Cycloalkenylen, $C_6$-$C_{12}$-Arylen, $C_7$-$C_{12}$-Aralkylen, oder einen zweiwertigen Rest der Formeln

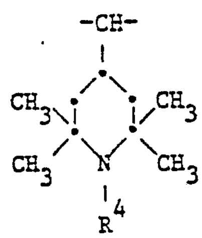

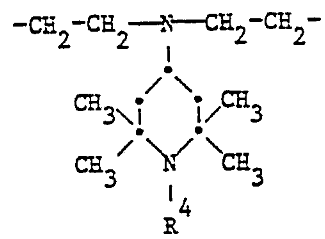

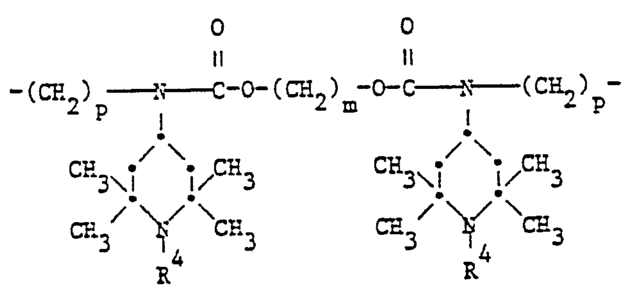

bedeutet, worin

p 1 oder 2 ist,

E Wasserstoff, $C_1$-$C_8$-Alkyl, Cyclohexyl, Benzyl oder eine Acylgruppe der Formel $R^7$—CO—, $R^8$—O—CO— oder $(R^9)(R^{10})$N—CO— und

E' eine Gruppe $R^{11}$O— oder $(R^{12})(R^{13})$N— darstellt,

worin

$R^3$ Wasserstoff, $C_1$-$C_6$-Alkyl, $C_2$-$C_7$-Alkoxymethyl, Phenoxymethyl oder Tolyloxymethyl bedeutet

$R^4$ Wasserstoff, $C_1$-$C_8$-Alkyl, $C_2$-$C_8$-Alkanoyl, $C_3$-$C_6$-Alkenoyl, $C_7$-$C_{12}$-Phenylalkyl oder $C_3$-$C_5$-Alkenylmethyl bedeutet,

$R^5$ Wasserstoff, $C_1$-$C_{12}$-Alkyl, Cyclohexyl, Phenyl, Benzyl, Phenylethyl, $C_2$-$C_8$-Alkanoyl, $C_3$-$C_6$-Alkenoyl oder Benzoyl bedeutet,

$R^6$ $C_2$-$C_{12}$-Alkylen, $C_4$-$C_8$-Alkenylen oder Xylylen bedeutet,

$R^7$ $C_1$-$C_{12}$-Alkyl, $C_2$-$C_5$-Alkenyl, $C_5$-$C_8$-Cycloalkyl, $C_6$-$C_{10}$-Aryl, $C_7$-$C_{12}$-Phenylalkyl oder eine Gruppe der Formel

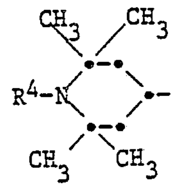

(IV)

bedeutet,

$R^8$ $C_1$-$C_{12}$-Alkyl, Allyl, Cyclohexyl oder Phenyl bedeutet,

$R^9$ Wasserstoff, $C_1$-$C_8$-Alkyl, Allyl, Cyclohexyl, Phenyl oder $C_7$-$C_{10}$-Alkylphenyl und

$R^{10}$ $C_1$-$C_8$-Alkyl, Allyl, Cyclohexyl, Phenyl oder $C_7$-$C_{10}$-Alkylphenyl bedeuten oder $R^9$ und $R^{10}$ zusammen mit dem N-Atom, an das sie gebunden sind, einen 5- bis 7-gliedrigen heterocyclischen Ring bilden,

$R^{11}$ $C_1$-$C_{12}$-Alkyl, $C_3$-$C_5$-Alkenylmethyl, $C_5$-$C_8$-Cycloalkyl, Phenyl, $C_7$-$C_{10}$-Alkylphenyl, $C_7$-$C_{12}$-Phenylalkyl, $C_3$-$C_{12}$-Alkoxyalkyl, eine Gruppe $-(CH_2CH_2O)_m-CH_3$, eine Gruppe der Formel IV oder eine Gruppe der Formel V

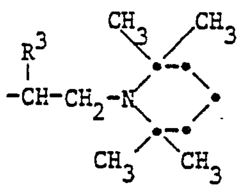

(V)

bedeutet,

$R^{12}$ $C_1$-$C_{12}$-Alkyl, Allyl, Cyclohexyl, Phenyl, Benzyl, $C_3$-$C_{12}$-Alkoxyalkyl, $C_4$-$C_{12}$-Dialkylaminoalkyl, eine Gruppe der Formel IV oder der Formel VI

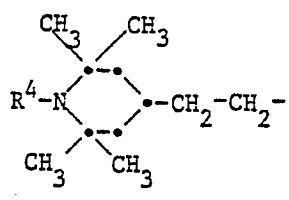

(VI)

bedeutet und

$R^{13}$ Wasserstoff, $C_1$-$C_{12}$-Alkyl, Allyl, Cyclohexyl oder eine Gruppe der Formel IV bedeutet, sowie deren Co-oligomere.

2. Oligomere gemäss Anspruch 1 der Formel I oder II, worin

n ein Wert von 1 bis 3 ist,

$R^1$ $C_2$-$C_{12}$-Alkylen, $C_4$-$C_{20}$-Mono- oder Polyoxa-alkylen oder eine Gruppe der Formel

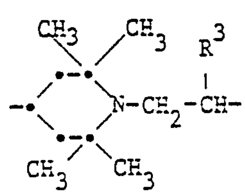

bedeutet,

$R^2$ $C_1$-$C_{12}$-Alkylen, $C_2$-$C_{12}$-Alkenylen oder Phenylen bedeutet,
E Wasserstoff oder eine Gruppe $R^7$—CO— und
E' eine Gruppe $R^{11}$O— ist,
$R^3$ Wasserstoff, $C_1$-$C_4$-Alkyl, $C_2$-$C_6$-Alkoxymethyl, Phenoxymethyl oder Tolyloxymethyl bedeutet,
$R^4$ Wasserstoff, $C_1$-$C_4$-Alkyl, Acetyl, Allyl oder Benzyl bedeutet,
$R^7$ $C_1$-$C_6$-Alkyl oder Phenyl und
$R^{11}$ $C_1$-$C_{12}$-Alkyl oder eine Gruppe der Formel IV oder V bedeutet.

3. Verfahren zur Herstellung von oligomeren Estern der Formel I,

$$E-\!\left(O-R^1-O-\overset{O}{\overset{\|}{C}}-R^2-\overset{O}{\overset{\|}{C}}\right)_{\!n}\!-O-R^1-O-E \qquad (I)$$

worin n $R^1$, $R^2$ und E die in Anspruch 1 gegebene Bedeutung haben, durch Umsetzung eines Diols HO—$R^1$—OH mit einem Dicarbonsäureester

$$R^{\circ}O-\overset{O}{\overset{\|}{C}}-R^2-\overset{O}{\overset{\|}{C}}-OR^{\circ}$$

worin R° $C_1$-$C_6$-Alkyl, Benzyl oder Phenyl bedeutet, in Gegenwart eines Umesterungskatalysators bei 80 bis 230 °C und gegebenenfalls anschliessende Umsetzung der Endgruppen mit einem monofunktionellen Reagens, dadurch gekennzeichnet, dass man die Ausgangskomponenten im Molverhältnis 1,33 : 1 bis 2 : 1 verwendet.

4. Verfahren zur Herstellung von oligomeren Estern der Formel II,

$$E'-\!\left(\overset{O}{\overset{\|}{C}}-R^2-\overset{O}{\overset{\|}{C}}-O-R^1-O\right)_{\!n}\!-\overset{O}{\overset{\|}{C}}-R^2-\overset{O}{\overset{\|}{C}}-E'$$

worin n, $R^1$, $R^2$ und E' die in Anspruch 1 gegebene Bedeutung haben, durch Umsetzung eines Diols HO—$R^1$—OH mit einem Dicarbonsäureester

$$R^{\circ}O-\overset{O}{\overset{\|}{C}}-R^2-\overset{O}{\overset{\|}{C}}-OR^{\circ}$$

worin R° $C_1$-$C_6$-Alkyl, Benzyl oder Phenyl bedeutet, in Gegenwart eines Umesterungskatalysators bei 80 bis 230 °C und gegebenenfalls anschliessende Umsetzung der Endgruppen mit einem monofunktionellen Reagens, dadurch gekennzeichnet, dass man die Ausgangskomponenten im Molverhältnis Diol : Dicarbonsäureester von 1 : 1,33 bis 1 : 2 verwendet.

5. Verfahren zur Herstellung von Co-oligomeren Estern der Formel I oder II gemäss Anspruch 3 oder 4, dadurch gekennzeichnet, dass man entweder zwei verschiedene Diole oder zwei verschiedene Dicarbonsäureester verwendet.

6. Verwendung der Verbindungen des Anspruches 1 als Lichtschutzmittel für organische Materialien.

7. Verwendung gemäss Anspruch 6 als Lichtschutzmittel für organische Polymere.

## Claims

1. An oligomeric ester of the formula I or II

$$E-\!\left(O-R^1-O-\overset{O}{\overset{\|}{C}}-R^2-\overset{O}{\overset{\|}{C}}\right)_{\!n}\!-O-R^1-O-E \qquad (I)$$

$$E'-\!\left(\overset{O}{\overset{\|}{C}}-R^2-\overset{O}{\overset{\|}{C}}-O-R^1-O\right)_{\!n}\!-\overset{O}{\overset{\|}{C}}-R^2-\overset{O}{\overset{\|}{C}}-E' \qquad (II)$$

in which
n has a value from 1 to 3,
$R^1$ and $R^2$ are divalent organic radicals, at least one of which contains a polyalkylpiperidine radical of the formula III

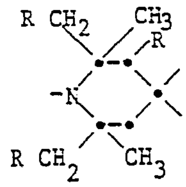

(III)

in which R is hydrogen or $C_1$-$C_4$-alkyl, whereby $R^1$ is $C_2$-$C_{18}$-alkylene, $C_4$-$C_8$-alkenylene, $C_4$-$C_{20}$-monooxaalkylene, $C_4$-$C_{20}$-polyoxaalkylene, $C_5$-$C_8$-cycloalkylene, $C_7$-$C_{10}$-cycloalkane-dialkylene, $C_8$-$C_{20}$-arene-dialkylene or a divalent radical of the formulae

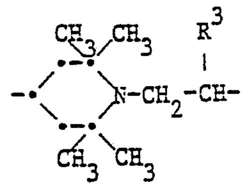

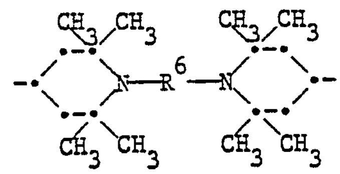

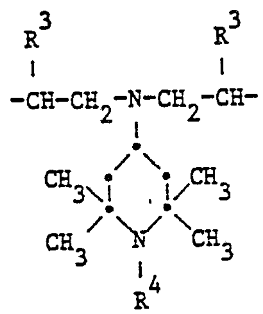

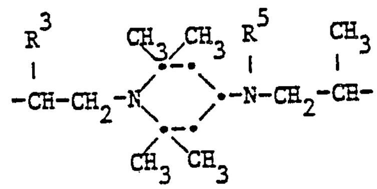

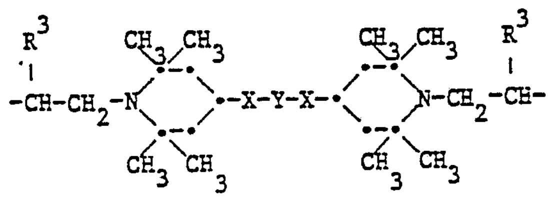

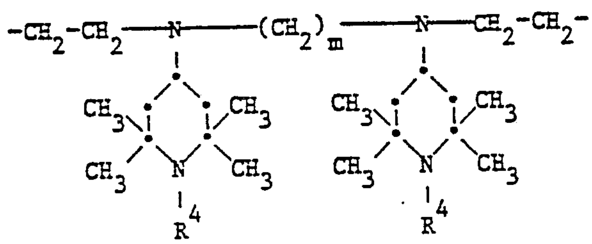

or

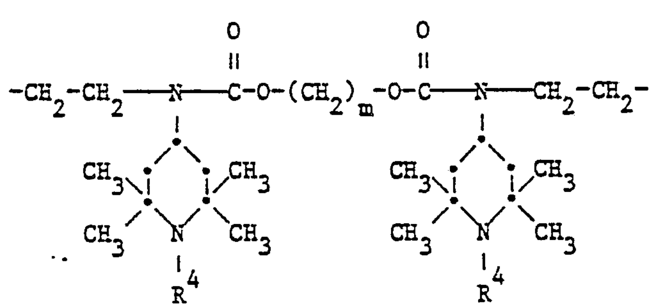

in which

m is 2 to 10,

X is a group —O— or —$NR^5$—,

Y is $C_2$-$C_{12}$-alkylene, $C_4$-$C_{12}$-alkylene which is interrupted by one or two —O— or —NH— groups, cyclohexylene, cyclohexane dimethylene, $C_6$-$C_{12}$-arylene

or -phenylene-Z-phenylene- in which

Z is —O—, —$CH_2$— or —$SO_2$—,

$R^2$ is a direct bond, $C_1$-$C_{18}$-alkylene, $C_2$-$C_6$-alkenylene, $C_5$-$C_8$-cycloalkylene, $C_5$-$C_8$-cycloalkenylene, $C_6$-$C_{12}$-arylene, $C_7$-$C_{12}$-aralkylene of a divalent radical of the formulae

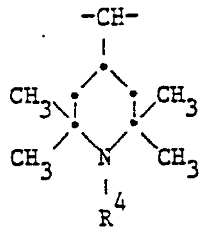

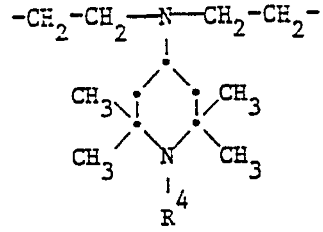

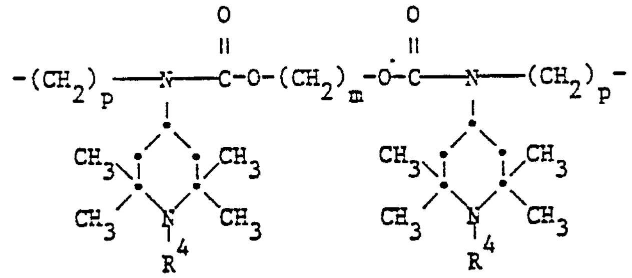

in which

p is 1 or 2,

E is hydrogen, $C_1$-$C_8$-alkyl, cyclohexyl, benzyl or an acyl group of the formula $R^7$—CO—, $R^8$—O—CO— or $(R^9)(R^{10})$N—CO— and

E' is a group $R^{11}$O— or $(R^{12})(R^{13})$N— and

wherein

$R^3$ is hydrogen, $C_1$-$C_6$-alkyl, $C_2$-$C_7$-alkoxymethyl, phenoxymethyl or tolyloxymethyl,

$R^4$ is hydrogen, $C_1$-$C_8$-alkyl, $C_2$-$C_8$-alkanoyl, $C_3$-$C_6$-alkenoyl, $C_7$-$C_{12}$-phenylalkyl or $C_3$-$C_5$-alkenylmethyl,

$R^5$ is hydrogen, $C_1$-$C_{12}$-alkyl, cyclohexyl, phenyl, benzyl, phenylethyl, $C_2$-$C_8$-alkanoyl, $C_3$-$C_6$-alkenoyl or benzoyl,

$R^6$ is $C_2$-$C_{12}$-alkylene, $C_4$-$C_8$-alkenylene or xylylene,

$R^7$ is $C_1$-$C_{12}$-alkyl, $C_2$-$C_5$-alkenyl, $C_5$-$C_8$-cycloalkyl, $C_6$-$C_{10}$-aryl, $C_7$-$C_{12}$-phenylalkyl or a group of the formula

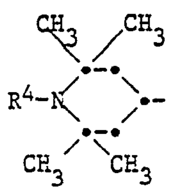

(IV)

$R^8$ is $C_1$-$C_{12}$-alkyl, allyl, cyclohexyl or phenyl,

$R^9$ is hydrogen, $C_1$-$C_8$-alkyl, allyl, cyclohexyl, phenyl or $C_7$-$C_{10}$-alkylphenyl and

$R^{10}$ is $C_1$-$C_8$-alkyl, allyl, cyclohexyl, phenyl or $C_7$-$C_{10}$-alkylphenyl, or $R^9$ and $R^{10}$, together with the N atom to which they are attached, form a 5-membered to 7-membered heterocyclic ring,

$R^{11}$ is $C_1$-$C_{12}$-alkyl, $C_3$-$C_5$-alkenylmethyl, $C_5$-$C_8$-cycloalkyl, phenyl, $C_7$-$C_{10}$-alkylphenyl, $C_7$-$C_{12}$-phenylalkyl, $C_3$-$C_{12}$-alkoxyalkyl, a group —$(CH_2CH_2O)_m$—$CH_3$, a group of the formula IV or a group of the formula V

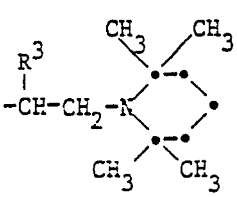

(V)

$R^{12}$ is $C_1$-$C_{12}$-alkyl, allyl, cyclohexyl, phenyl, benzyl, $C_3$-$C_{12}$-alkoxyalkyl, $C_4$-$C_{12}$-dialkylaminoalkyl, a group of the formula IV or a group of the formula VI

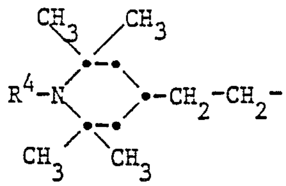

(VI)

and

$R^{13}$ is hydrogen, $C_1$-$C_{12}$-alkyl, allyl, cyclohexyl or a group of the formula IV, and co-oligomers thereof.

2. An oligomer according to Claim 1 of the formula I or II in which

n has a value from 1 to 3,

$R^1$ is $C_2$-$C_{12}$-alkylene, $C_4$-$C_{20}$-monooxaalkylene, $C_4$-$C_{20}$-polyoxaalkylene or a group of the formula

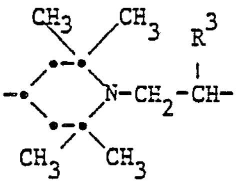

$R^2$ is $C_1$-$C_{12}$-alkylene, $C_2$-$C_{12}$-alkenylene or phenylene,

E is hydrogen or a group $R^7$—CO— and

E' is a group $R^{11}$O—,

$R^3$ is hydrogen, $C_1$-$C_4$-alkyl, $C_2$-$C_6$-alkoxymethyl, phenoxymethyl or tolyloxymethyl,

$R^4$ is hydrogen, $C_1$-$C_4$-alkyl, acetyl, allyl or benzyl,

$R^7$ is $C_1$-$C_6$-alkyl or phenyl and

$R^{11}$ is $C_1$-$C_{12}$-alkyl or a group of the formula IV or V.

3. A process for the preparation of an oligomeric ester of the formula I

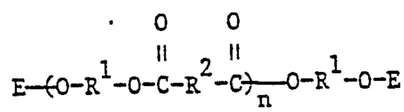

(I)

in which n, $R^1$, $R^2$ and E have the meaning given in Claim 1, by reacting a diol HO—$R^1$—OH with a dicarboxylic acid ester

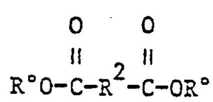

in which R° is $C_1$-$C_6$-alkyl, benzyl or phenyl, in the presence of a transesterification catalyst at 80 to 230 °C, and, if appropriate, subsequently reacting the end groups with a monofunctional reagent, which comprises using the starting components in a molar ratio of 1.33 : 1 to 2 : 1.

4. A process for the preparation of an oligomeric ester of the formula II

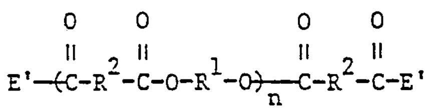

(II)

in which n, $R^1$, $R^2$ and E' have the meaning given in Claim 1, by reacting a diol HO—$R^1$—OH with a dicarboxylic acid ester

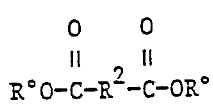

in which R° is $C_1$-$C_6$-alkyl, benzyl or phenyl, in the presence of a transesterification catalyst at 80 to 230 °C, and, if appropriate, subsequently reacting the end groups with a monofunctional reagent, which comprises using the starting components in a molar ratio diol : dicarboxylic acid ester of 1 : 1.33 to 1 : 2.

5. A process for the preparation of a co-oligomeric ester of the formula I or II according to Claim 3 or

4, wherein either two different diols or two different dicarboxylic acid esters are used.

6. The use of a compound of Claim 1 as a light stabiliser for organic material.

7. Use according to Claim 6 as a light stabiliser for organic polymers.

**Revendications**

1. Esters oligomères répondant à l'une des formules I et II :

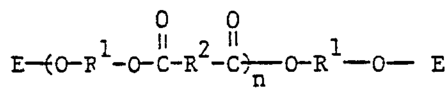

(I)

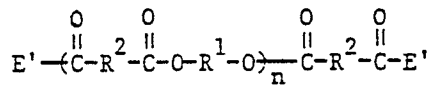

(II)

dans lesquelles :

n représente un nombre de 1 à 3,

$R^1$ et $R^2$ représentent des radicaux organiques bivalents dont au moins un contient un radical polyalkyl-pipéridinique répondant à la formule III :

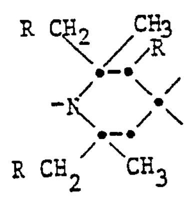

(III)

dans laquelle R représente l'hydrogène ou un alkyle en $C_1$-$C_4$, plus précisément $R^1$ représente un alkylène ,en $C_2$-$C_{18}$, un alcénylène en $C_4$-$C_8$, un mono-oxa- ou polyoxa-alkylène en $C_4$-$C_{20}$, un cycloalkylène en $C_5$-$C_8$, un cycloalcane-dialkylène en $C_7$-$C_{10}$, un arène-dialkylène en $C_8$-$C_{20}$ ou un radical bivalent répondant à l'une des formules suivantes :

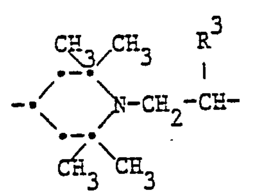

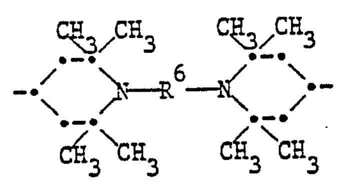

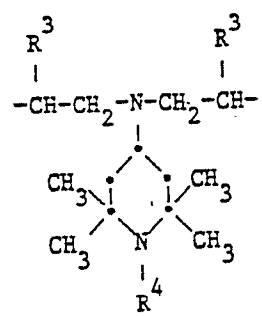

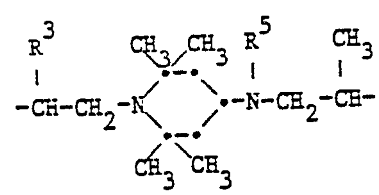

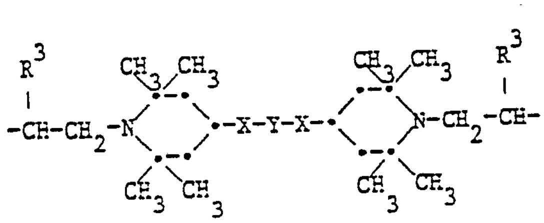

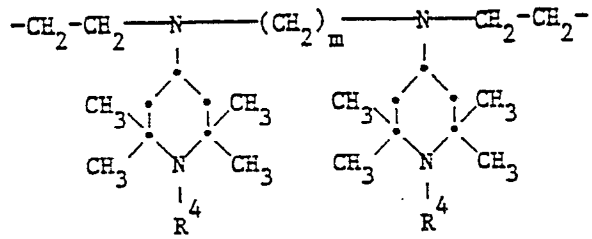 et

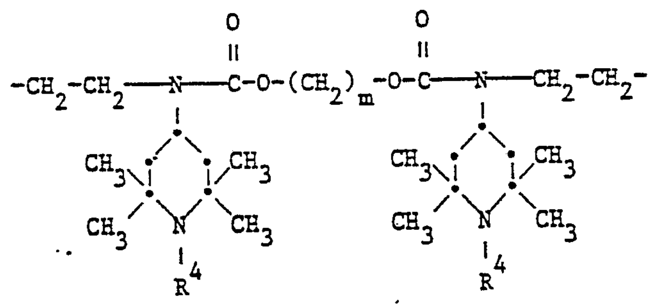

m désignant un nombre de 2 à 10,

X représente un radical —O— ou —$NR^5$—,

Y représente un alkylène en $C_2$-$C_{12}$, un alkylène en $C_4$-$C_{12}$ interrompu par un ou deux radicaux —O— ou —NH—, un cyclohexylène, un cyclohexane-diméthylène, un arylène en $C_6$-$C_{12}$ ou un radical -phénylène-Z-phénylène- dont le symbole

Z représente un pont —O—, —$CH_2$— ou —$SO_2$—,

$R^2$ représente une liaison directe, un alkylène en $C_1$-$C_{18}$, un alcénylène en $C_2$-$C_6$, un cycloalkylène en $C_5$-$C_8$, un cycloalcénylène en $C_5$-$C_8$, un arylène en $C_6$-$C_{12}$, un aralkylène en $C_7$-$C_{12}$ ou un radical bivalent répondant à l'une des formules suivantes :

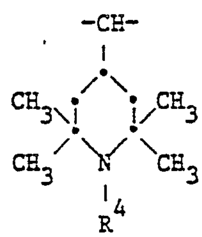

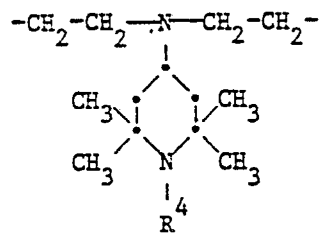

et

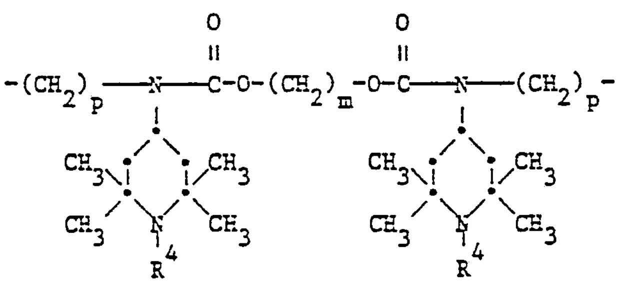

p est égal à 1 ou à 2,

E représente l'hydrogène, un alkyle en $C_1$-$C_8$, un cyclohexyle, un benzyle ou un acyle de formule $R^7$—CO—, $R^8$—O—CO— ou $(R^9)(R^{10})$N—CO—,

E' représente un radical $R^{11}$O— ou $(R^{12})(R^{13})$N—,

$R^3$ représente l'hydrogène, un alkyle en $C_1$-$C_6$, un alcoxyméthyle en $C_2$-$C_7$, un phénoxyméthyle ou un tolyloxyméthyle,

$R^4$ représente l'hydrogène, un alkyle en $C_1$-$C_8$, un alcanoyle en $C_2$-$C_8$, un alcénoyle en $C_3$-$C_6$, un phénylalkyle en $C_7$-$C_{12}$ ou un alcénylméthyle en $C_3$-$C_5$,

$R^5$ représente l'hydrogène, un alkyle en $C_1$-$C_{12}$, un cyclohexyle, un phényle, un benzyle, un phényléthyle, un alcanoyle en $C_2$-$C_8$, un alcénoyle en $C_3$-$C_6$ ou un benzoyle,

$R^6$ représente un alkylène en $C_2$-$C_{12}$, un alcénylène en $C_4$-$C_8$ ou un xylylène,

$R^7$ représente un alkyle en $C_1$-$C_{12}$, un alcényle en $C_2$-$C_5$, un cycloalkyle en $C_5$-$C_8$, un aryle en $C_6$-$C_{10}$, un phénylalkyle en $C_7$-$C_{12}$ ou un radical de formule IV :

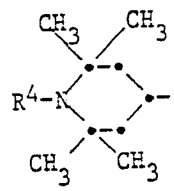

(IV)

$R^8$ représente un alkyle en $C_1$-$C_{12}$, un allyle, un cyclohexyle ou un phényle,

$R^9$ représente l'hydrogène, un alkyle en $C_1$-$C_8$, un allyle, un cyclohexyle, un phényle ou un alkylphényle en $C_7$-$C_{10}$,

$R^{10}$ représente un alkyle en $C_1$-$C_8$, un allyle, un cyclohexyle, un phényle ou un alkylphényle en $C_7$-$C_{10}$, ou encore $R^9$ et $R^{10}$ forment ensemble, et avec l'atome d'azote auquel ils sont liés, un hétérocycle comportant de 5 à 7 maillons,

$R^{11}$ représente un alkyle en $C_1$-$C_{12}$, un alcénylméthyle en $C_3$-$C_5$, un cycloalkyle en $C_5$-$C_8$, un phényle, un alkylphényle en $C_7$-$C_{10}$, un phénylalkyle en $C_7$-$C_{12}$, un alcoxyalkyle en $C_3$-$C_{12}$, un radical $—(CH_2CH_2O)_m—CH_3$, un radical de formule IV ou un radical de formule V :

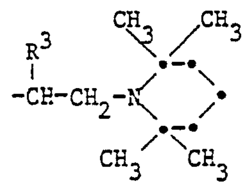

(V)

$R^{12}$ représente un alkyle en $C_1$-$C_{12}$, un allyle, un cyclohexyle, un phényle, un benzyle, un alcoxyalkyle en $C_3$-$C_{12}$, un dialkylamino-alkyle en $C_4$-$C_{12}$, un radical de formule IV ou un radical de formule VI :

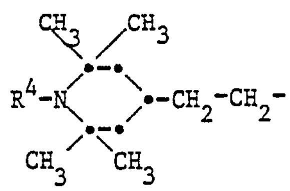

(VI)

et

$R^{13}$ représente l'hydrogène, un alkyle en $C_1$-$C_{12}$, un allyle, un cyclohexyle ou un radical de formule IV,

ainsi que les co-oligomères correspondants.

2. Oligomères de formule I ou II selon, la revendication 1, dans lesquels :

n représente un nombre de 1 à 3,

$R^1$ représente un alkylène en $C_2$-$C_{12}$, un mono-oxa- ou polyoxa-alkylène en $C_4$-$C_{20}$ ou un radical de formule :

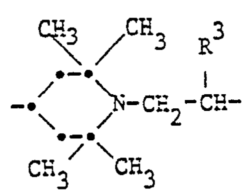

$R^2$ représente un alkylène en $C_1$-$C_{12}$, un alcénylène en $C_2$-$C_{12}$ ou un phénylène,

E représente l'hydrogène ou un radical $R^7—CO—$,

E' représente un radical $R^{11}O—$,

$R^3$ représente l'hydrogène, un alkyle en $C_1$-$C_4$, un alcoxyméthyle en $C_2$-$C_6$, un phénoxyméthyle ou un tolyloxyméthyle,

$R^4$ représente l'hydrogène, un alkyle en $C_1$-$C_4$, un acétyle, un allyle ou un benzyle,

$R^7$ représente un alkyle en $C_1$-$C_6$ ou un phényle et

$R^{11}$ représente un alkyle en $C_1$-$C_{12}$ ou un radical de formule IV ou V.

3. Procédé de préparation d'esters oligomères répondant à la formule I :

$$E-(O-R^1-O-\overset{\overset{O}{\|}}{C}-R^2-\overset{\overset{O}{\|}}{C})_n-O-R^1-O-E \qquad (I)$$

dans laquelle n, $R^1$, $R^2$ et E ont les significations données à la revendication 1, par réaction d'un diol HO—$R^1$—OH avec un ester d'acide dicarboxylique

$$R^\circ O-\overset{\overset{O}{\|}}{C}-R^2-\overset{\overset{O}{\|}}{C}-OR^\circ$$

dans lequel R° représente un alkyle en $C_1$-$C_6$, un benzyle ou un phényle, en présence d'un catalyseur de transestérification, à une température de 80 à 230 °C, puis, éventuellement, réaction des radicaux terminaux avec un réactif monofonctionnel, procédé caractérisé en ce qu'on utilise les corps de départ dans un rapport molaire compris entre 1,33 : 1 et 2 : 1.

4. Procédé de préparation d'esters oligomères répondant à la formule II :

$$E'-(\overset{\overset{O}{\|}}{C}-R^2-\overset{\overset{O}{\|}}{C}-O-R^1-O)_n-\overset{\overset{O}{\|}}{C}-R^2-\overset{\overset{O}{\|}}{C}-E' \qquad (II)$$

dans laquelle n, $R^1$, $R^2$ et E ont les significations données à la revendication 1, par réaction d'un diol HO—$R^1$—OH avec un ester d'acide dicarboxylique

$$R^\circ O-\overset{\overset{O}{\|}}{C}-R^2-\overset{\overset{O}{\|}}{C}-OR^\circ$$

dans lequel R° représente un alkyle en $C_1$-$C_6$, un benzyle ou un phényle, en présence d'un catalyseur de transestérification, à une température de 80 à 230 °C, puis, éventuellement, réaction des radicaux terminaux avec un réactif monofonctionnel, procédé caractérisé en ce qu'on utilise les corps de départ dans un rapport molaire (diol : ester d'acide dicarboxylique) compris entre 1 : 1,33 et 1 : 2.

5. Procédé de préparation d'esters co-oligomères de formule I ou II selon l'une des revendications 3 et 4, procédé caractérisé en ce qu'on utilise soit deux diols différents, soit deux esters d'acides dicarboxyliques différents.

6. Application des composés de la revendication 1 comme stabilisants à la lumière pour des matières organiques.

7. Application selon la revendication 6 caractérisée en ce que les composés sont utilisés comme stabilisants à la lumière pour des polymères organiques.